# EUROPEAN PATENT APPLICATION

(11) **EP 4 763 255 A1**
(43) Date of publication of application: **24.06.2026**
(21) Application number: 25224731.7
(22) Date of filing: 18.12.2025
(51) Int. Cl.: A61M 16/00, G01N 29/00, G01N 29/02

(54) **RESPIRATORY SUPPORT DEVICE AND METHOD FOR DETECTING OXYGEN CONCENTRATION IN RESPIRATORY SUPPORT DEVICE**

(30) Priority: 19.12.2024 CN 202411899072
(71) Applicant: Shenzhen Mindray Bio-Medical Electronics Co., Ltd., Shenzhen, Guangdong 518057 (CN)
(72) Inventor: LIU, Jinglei, Shenzhen, 518057 (CN); WANG, Chao, Shenzhen, 518057 (CN); ZHANG, Zhe, Shenzhen, 518057 (CN)
(74) Representative: KIPA AB

(57) **Abstract**

According to a respiratory support device and method for detecting an oxygen concentration in the respiratory support device provided in the application, a gas characteristic of a gas in a respiratory gas flow path and/or a device parameter of the respiratory support device are acquired first; then a target oxygen concentration detection value is determined based on the gas characteristic and/or the device parameter, and the target oxygen concentration detection value is outputted optionally, the target oxygen concentration detection value is obtained based on an oxygen concentration detection value of an ultrasonic oxygen sensor at a target time or in a target time interval where an interference of an interference gas with the oxygen concentration detection meets a preset condition. Therefore, the oxygen concentration detection value which is not affected by the interference is obtained, thereby improving detection accuracy.

## Description

### TECHNICAL FIELD

The application relates to the field of medical devices, and in particular to a respiratory support device and a method for detecting an oxygen concentration in the respiratory support device.

### BACKGROUND

An existing respiratory support device such as a ventilator, an anesthesia machine or the like, usually uses an oxygen sensor to monitor an oxygen concentration in a gas path, and for example, the oxygen sensor uses an oxygen cell or a paramagnetic oxygen sensor. The oxygen cell is a consumable with a short life (1 year), which brings a problem of high maintenance cost; the paramagnetic oxygen sensor is not resistant to vibration, which brings a problem of an inaccurate oxygen concentration after it is subject to vibration or impact.

Furthermore, an ultrasonic oxygen sensor is a non-consumable with a long life (10 years), is resistant to vibration, and does not have these shortcomings of the oxygen cell and the paramagnetic oxygen sensor. However, accuracy of an oxygen concentration monitoring value of a respiratory support device using the ultrasonic oxygen sensor is usually not as good as that of an oxygen concentration monitoring value of a respiratory support device using the oxygen cell or the paramagnetic oxygen sensor. If the accuracy of the oxygen concentration monitoring value may be improved, it may be helpful for application of the ultrasonic oxygen sensor in the respiratory support device, and market competitiveness of such product may be significantly improved.

Therefore, accuracy of an oxygen concentration detection value of the existing respiratory support device using the ultrasonic oxygen sensor still needs to be improved.

### SUMMARY

The application mainly provides a respiratory support device and a method for detecting an oxygen concentration in the respiratory support device, which are intended to improve the accuracy of the oxygen concentration detection value.

An embodiment provides a respiratory support device, the respiratory support device includes a respiratory gas flow path, an ultrasonic oxygen sensor, and a processor.

The respiratory gas flow path is configured to connect to a patient and deliver an oxygen-containing gas to the patient during a treatment time period to provide respiratory support, the respiratory gas flow path further optionally contains an interference gas that includes at least part of an exhaled gas by the patient.

The ultrasonic oxygen sensor is configured to detect an oxygen concentration of a gas in the respiratory gas flow path, the gas in the respiratory gas flow path includes at least the oxygen-containing gas and optionally the interference gas.

The processor is configured to:
acquire a gas characteristic of the gas in the respiratory gas flow path and/or a device parameter of the respiratory support device; and
determine a target oxygen concentration detection value based on the gas characteristic and/or the device parameter, and optionally output the target oxygen concentration detection value, here the target oxygen concentration detection value is obtained based on an oxygen concentration detection value of the ultrasonic oxygen sensor at a target time or in a target time interval where an interference of the interference gas with the oxygen concentration detection meets a preset condition.

An embodiment provides a respiratory support device, the respiratory support device includes a respiratory gas flow path, an ultrasonic oxygen sensor, a buffer member or an isolation member, and a processor.

The respiratory gas flow path is configured to connect to a patient and deliver an oxygen-containing gas to the patient to provide respiratory support, the respiratory gas flow path further optionally contains an interference gas.

The ultrasonic oxygen sensor is configured to detect an oxygen concentration of a gas in the respiratory gas flow path, the gas in the respiratory gas flow path includes at least the oxygen-containing gas and optionally the interference gas.

The buffer member or the isolation member is arranged at a front end or a rear end of the ultrasonic oxygen sensor, and is configured to reduce or block the interference gas from entering into a detection range of the ultrasonic oxygen sensor.

The processor is configured to acquire an oxygen concentration detection value of the gas in the respiratory gas flow path detected by the ultrasonic oxygen sensor, determine a target oxygen concentration detection value based on the oxygen concentration detection value, and output the target oxygen concentration detection value.

An embodiment provides a method for detecting an oxygen concentration in a respiratory support device, the respiratory support device includes a respiratory gas flow path and an ultrasonic oxygen sensor. The respiratory gas flow path is configured to connect to a patient and deliver an oxygen-containing gas to the patient during a treatment time period to provide respiratory support, the respiratory gas flow path further optionally contains an interference gas that includes at least part of an exhaled gas by the patient. The ultrasonic oxygen sensor is configured to detect an oxygen concentration of a gas in the respiratory gas flow path, the gas in the respiratory gas flow path includes at least the oxygen-containing gas and optionally the interference gas. The method includes the following operations.

A gas characteristic of the gas in the respiratory gas flow path and/or a device parameter of the respiratory support device are acquired.

A target oxygen concentration detection value is determined based on the gas characteristic and/or the device parameter, and the target oxygen concentration detection value is outputted optionally, the target oxygen concentration detection value is obtained based on an oxygen concentration detection value of the ultrasonic oxygen sensor at a target time or in a target time interval where an interference of the interference gas with the oxygen concentration detection meets a preset condition.

An embodiment provides a computer-readable storage medium, the medium has stored thereon a program, the program may be executed by a processor to implement the method as described above.

According to the respiratory support device and the method for detecting an oxygen concentration in the respiratory support device provided in the above embodiments, a gas characteristic of the gas in the respiratory gas flow path and/or a device parameter of the respiratory support device are acquired first; then a target oxygen concentration detection value is determined based on the gas characteristic and/or the device parameter, and the target oxygen concentration detection value is outputted optionally, the target oxygen concentration detection value is obtained based on an oxygen concentration detection value of the ultrasonic oxygen sensor at a target time or in a target time interval where an interference of the interference gas with the oxygen concentration detection meets a preset condition. Therefore, the oxygen concentration detection value which is not affected by the interference is obtained, thereby improving detection accuracy.

### BRIEF DESCRIPTION OF THE DRAWINGS

FIG. 1 is a structural block diagram of an embodiment of a respiratory support device provided in the application.
FIG. 2 is a schematic diagram of an airflow direction in an exhalation stage in the respiratory support device provided in the application.
FIG. 3 is a schematic diagram of an airflow direction in an inhalation stage in the respiratory support device provided in the application.
FIG. 4 is a flowchart of an embodiment of a method for detecting an oxygen concentration provided in the application.
FIG. 5 is a flowchart of an embodiment of an operation 2 in FIG. 4.
FIG. 6 is a flowchart of an embodiment of an operation 21 in FIG. 5.
FIG. 7 shows a variation curve of oxygen concentration interfered by an interference gas, and a variation curve of flow rate in the related art.
FIG. 8 is a flowchart of another embodiment of operation 2 in FIG. 4.
FIG. 9 is a structural block diagram of another embodiment of the respiratory support device provided in the application.
FIG. 10 is a schematic diagram of a gas path where a buffer gas path is provided in the respiratory support device provided in the application.
FIG. 11 is a schematic diagram of a gas path where an on/off valve is provided in the respiratory support device provided in the application.

### DETAILED DESCRIPTION

[**0033]** The application will be further described in detail below with reference to specific implementations in combination with the drawings. Similar elements in different implementations use associated similar element reference numerals. In the following implementations, many details are described to enable the application to be understood better. However, it may be effortlessly recognized by those skilled in the art that some of features may be omitted in different situations or may be replaced by other elements, materials and methods. In some situations, some operations relevant to the application are not shown or described in the description to avoid core parts of the application from being overwhelmed by excessive descriptions, it is unnecessary for those skilled in the art to describe these relevant operations in detail, and these relevant operations may be fully understood by those skilled in the art based on descriptions in the description and common technical knowledge in the art.

[**0034]** Furthermore, characteristics, operations or features described in the description may be combined in any suitable manner, to form various implementations. Meanwhile, sequences of steps or actions in descriptions of the method may also be exchanged or adjusted in a manner apparent to those skilled in the art. Therefore, various sequences in the description and drawings are only for the purpose of describing a certain embodiment clearly, and do not imply necessary sequences, unless otherwise indicated that a certain sequence must be followed.

[**0035]** Serial numbers assigned to components here, such as "first", "second" or the like, themselves are only intended to distinguish described objects and do not have any meaning of sequence or technology. Furthermore, "connect" and "couple" described in the application include direct and indirect connection (coupling), unless otherwise specified.

[**0036]** The application may improve accuracy of oxygen concentration detection of a respiratory support device in various manners, for example, by determining a time or a time interval where an ultrasonic oxygen sensor is not interfered by an interference gas, using an oxygen concentration acquired by the ultrasonic oxygen sensor at the time or the time interval as a target oxygen concentration, and outputting the target oxygen concentration; for another example, by improving a gas path relevant to the ultrasonic oxygen sensor such that the interference gas does not pass through the ultrasonic oxygen sensor, thereby improving the accuracy of oxygen concentration detection. These contents will be described in detail below by some embodiments.

[**0037]** In general, conventional respiratory support devices in the art include a single-tube ventilator and a double-tube ventilator. The double-tube ventilator refers to a ventilator which is provided with two pipelines for an inhalation port and an exhalation port simultaneously, and is commonly used in an intensive care ventilator, etc. The double-tube ventilator is provided with an inhalation valve and an exhalation valve simultaneously. A patient is supplied with air in an inhalation flow path by opening the inhalation valve, and the patient is helped to exhale in an exhalation flow path by opening the exhalation valve. Therefore, an inhaled tidal volume and an exhaled tidal volume by the patient may be measured accurately, and such structure may provide a more complex ventilation mode, the patient's more monitoring parameters, a better stability and reliability.

[**0038]** The single-tube ventilator refers to a ventilator which uses the same pipeline for inhalation and exhalation, and is commonly used in a non-invasive ventilator, an oxygen therapy device, etc. The single-tube ventilator supplies air to the patient through a built-in inhalation valve and helps the patient to exhale through an external exhalation valve. The single-tube ventilator usually has a simpler structure, and provides relatively fewer ventilation modes and monitoring parameters than the double-tube ventilator, and thus is used for patients with mild symptoms more commonly. Since the same pipeline of the single-tube ventilator is used for inhalation and exhalation, oxygen concentration detection thereof is interfered by the interference gas more easily.

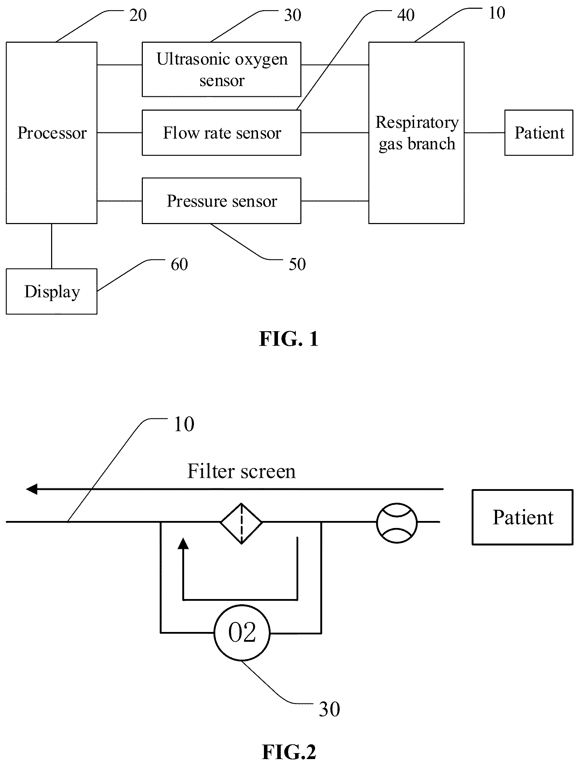

[**0039]** As shown in FIG. 1 to FIG. 3, a respiratory support device provided in the application includes a respiratory gas flow path 10, a processor 20 and an ultrasonic oxygen sensor (an ultrasonic wave-type oxygen sensor) 30.

[**0040]** The respiratory gas flow path 10 is configured to connect to a patient, and deliver an oxygen-containing gas to the patient during a treatment time period to provide respiratory support. The respiratory gas flow path 10 is communicated with the patient's respiratory tract, and when the patient breathes non-invasively, the respiratory gas flow path 10 may also be communicated with the outside. An exhaled gas by the patient, gases from the outside or the like may cause interference to the ultrasonic oxygen sensor 30 when they enter the respiratory gas flow path 10. That is, this type of interference gas may enter the respiratory gas flow path 10, that is, the respiratory gas flow path 10 further optionally contains an interference gas. The interference gas includes one or more of: the exhaled gas by the patient, nebulized medication introduced into the respiratory gas flow path 10 driven by the exhaled gas, and water vapor introduced into the respiratory gas flow path 10 driven by the exhaled gas. The interference gas is mainly the exhaled gas by the patient, the interference gas includes at least part of the exhaled gas by the patient, and the exhaled gas by the patient includes carbon dioxide and water vapor, etc.

[0041] The ultrasonic oxygen sensor 30 is configured to detect an oxygen concentration of a gas in the respiratory gas flow path 10, the gas in the respiratory gas flow path 10 includes at least the oxygen-containing gas and optionally the interference gas. The ultrasonic oxygen sensor 30 may be directly arranged inside the respiratory gas flow path 10, or may be communicated with the respiratory gas flow path 10, and in short, as long as the ultrasonic oxygen sensor 30 may detect the oxygen concentration of the gas in the respiratory gas flow path 10.

[**0042]** The oxygen concentration of the gas in the respiratory gas flow path 10 as mentioned above usually refers to an oxygen concentration of a gas output from the respiratory support device or an oxygen concentration of an inhaled gas by the patient. Since the single-tube ventilator does not distinguish an inhalation flow path from an exhalation flow path clearly, it is more likely for the exhaled gas by the patient to mix with the gas output from the respiratory support device, thereby interfering with the detected oxygen concentration. Therefore, the application is particularly applicable to the single-tube ventilator.

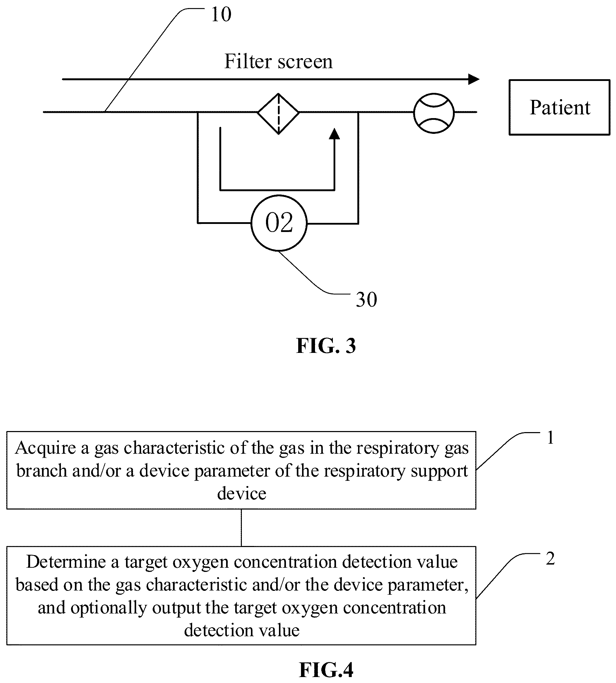

[**0043]** The processor 20 is configured to perform a method for detecting an oxygen concentration, to improve accuracy of detecting the oxygen concentration by using the ultrasonic oxygen sensor 30. A specific process is shown in FIG. 4, and includes the following operations 1 and 2.

[**0044]** In operation 1, the processor 20 acquires a gas characteristic of the gas in the respiratory gas flow path 10 and/or a device parameter of the respiratory support device. The respiratory support device may be any device capable of providing respiratory support to the patient, such as a ventilator, an anesthesia machine, an oxygen therapy device or the like, and the ventilator may include a double-tube ventilator and a single-tube ventilator. Considering that oxygen concentration detection of the single-tube ventilator is interfered by the interference gas more easily, embodiments are explained by an example that the respiratory support device includes a single-tube ventilator, for example, the respiratory support device may be a single-tube ventilator or a device including the single-tube ventilator. The respiratory support device may further include a flow rate sensor 40 and/or a pressure sensor 50. The gas characteristic of the gas in the respiratory gas flow path 10 may be obtained by one or more of the flow rate sensor 40, the pressure sensor 50 and the ultrasonic oxygen sensor 30. Specifically, in an embodiment, the gas characteristic acquired by the processor 20 may include one or more of: the oxygen concentration detection value of the gas in the respiratory gas flow path 10 detected by the ultrasonic oxygen sensor 30, a flow rate detection value of a gas flowing through the ultrasonic oxygen sensor 30 detected by the ultrasonic oxygen sensor 30 (some ultrasonic oxygen sensors have a function of detecting the flow rate), a flow direction of the gas in the respiratory gas flow path 10 detected by the flow rate sensor 40, a flow rate detection value of a gas flowing through the ultrasonic oxygen sensor 30 detected by the flow rate sensor 40, a flow rate detection value of the gas in the respiratory gas flow path 10 detected by the flow rate sensor 40, and a pressure detection value of the gas in the respiratory gas flow path 10 detected by the pressure sensor 50.

[**0045]** The device parameter acquired by the processor 20 may include one or more of: a respiratory rate, a respiratory ratio and an output flow rate of the oxygen-containing gas set by the respiratory support device.

[**0046]** For example, the respiratory support device is a ventilator, and usually, device parameters thereof may further include an oxygen content of the oxygen-containing gas, an inhalation pressure, a tidal volume, etc. These device parameters may be set and adjusted by a user.

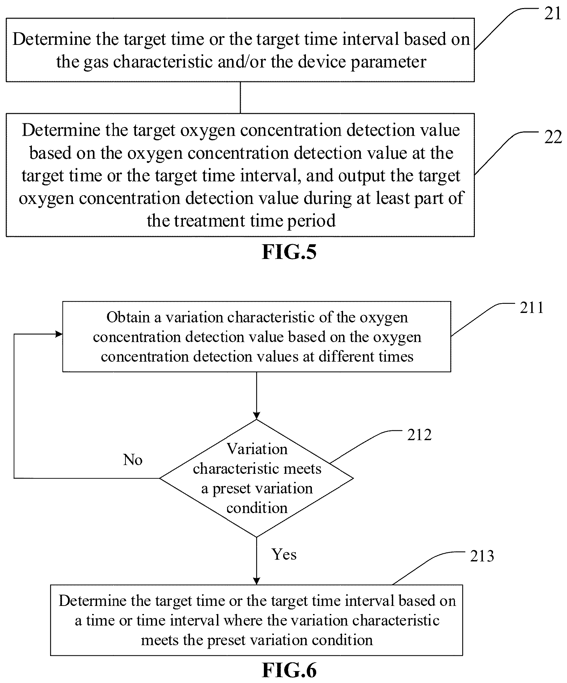

[**0047]** In operation 2, the processor 20 determines a target oxygen concentration detection value based on the gas characteristic and/or the device parameter, and optionally outputs the target oxygen concentration detection value. The target oxygen concentration detection value is obtained based on an oxygen concentration detection value of the ultrasonic oxygen sensor 30 at a target time or in a target time interval where an interference of the interference gas with the oxygen concentration detection meets a preset condition. Specifically, as shown in FIG. 5, the operation 2 may include operations 21 and 22. In operation 21, the processor 20 may determine the target time or the target time interval based on the gas characteristic and/or the device parameter. In operation 22, the processor 20 may determine the target oxygen concentration detection value based on the oxygen concentration detection value at the target time or in the target time interval, and output the target oxygen concentration detection value during at least part of the treatment time period. The at least part of the treatment time period includes the target time or at least part of the target time interval, and other time(s) or at least part(s) of other time interval(s) than the target time or the target time interval. This process may be performed by the processor 20 in real time, thereby outputting the target oxygen concentration detection value in real time. The respiratory support device may further include a display 60, the processor 20 outputs the target oxygen concentration detection value, and specifically, the processor 20 may output the target oxygen concentration detection value to the display 60 to display it, such that the user may know the oxygen concentration of the inhaled gas by the patient in real time. Of course, the processor 20 may also output the target oxygen concentration detection value to other devices (such as a monitor, etc.).

[**0048]** The target time or the target time interval may be a time or a time interval where the ultrasonic oxygen sensor 30 is substantially not interfered by the interference gas, such that the obtained target oxygen concentration detection value is very accurate.

[**0049]** Of course, oxygen concentration data detected by the ultrasonic oxygen sensor 30 at other time(s) than the target time or the target time interval is not completely useless. In some embodiments, the processor 20 may correct an oxygen concentration detection value of the ultrasonic oxygen sensor 30 at other time(s) or in at least part(s) of other time interval(s) than the target time or the target time interval based on the gas characteristic and/or the device parameter, and output a corrected oxygen concentration detection value.

[**0050]** One of main reasons limiting application of the ultrasonic oxygen sensor in the respiratory support device such as the ventilator is that detection accuracy of the ultrasonic oxygen sensor is easily affected by the interference gas. The ultrasonic oxygen sensor is often used for monitoring oxygen concentrations of binary gases. Common binary gases are air and oxygen. When there are other interference gases than air and oxygen, the accuracy of oxygen concentration detection cannot be guaranteed. Common interference gases in the ventilator are CO₂ and water vapor. CO₂ comes from the exhaled gas by the patient, and the water vapor comes from air inhaled by the ventilator from an ambient environment, the exhaled gas by the patient, etc. In view of influence of the interference gas, a commonly used solution at present is to add a sensor to measure a content of the interference gas, and then compensate an output value of the ultrasonic oxygen sensor based on the content of the interference gas. However, the solution has a complex compensation algorithm while needs to introduce an additional sensor, and when types of interference gases increase, compensation difficulty also increases sharply. Other solutions are to monitor the oxygen concentration by using an oxygen sensor which is not affected by the interference gas, such as an oxygen cell or a paramagnetic oxygen sensor. However, the oxygen cell is a consumable with a short life (1 year), which brings a problem of high maintenance cost; the paramagnetic oxygen sensor is not resistant to vibration, which brings a problem of an inaccurate oxygen concentration after it is subject to vibration or impact. The ultrasonic oxygen sensor is a non-consumable with a long life (10 years), is resistant to vibration, and is more competitive than other sensors. The application solves the influence of the interference gas on the ultrasonic oxygen sensor by the above means, which is helpful for application of the ultrasonic oxygen sensor in the ventilator, and may significantly improve market competitiveness of the ventilator using the ultrasonic oxygen sensor.

[**0051]** There may be multiple specific processing modes for the processor 20 to determine the target oxygen concentration detection value based on the gas characteristic and/or the device parameter, which will be described below respectively.

[**0052]** In an embodiment where a first processing mode is used, the processor 20 determines the target oxygen concentration detection value under the first processing mode based on the gas characteristic and/or the device parameter, and outputs the target oxygen concentration detection value. For example, the gas characteristic includes an oxygen concentration detection value of the gas in the respiratory gas flow path 10 detected by the ultrasonic oxygen sensor 30. The processor 20 may determine the target time or the target time interval based on the oxygen concentration detection value of the gas in the respiratory gas flow path 10 detected by the ultrasonic oxygen sensor 30, determine the target oxygen concentration detection value based on the oxygen concentration detection value at the target time or in the target time interval, and output the target oxygen concentration detection value during at least part of the treatment time period.

[**0053]** Specifically, as shown in FIG. 6, the operation 21 may specifically include the following operations 211 to 213.

[**0054]** In operation 211, the processor 20 obtains a variation characteristic of the oxygen concentration detection value based on the oxygen concentration detection values at different times.

[**0055]** In operation 212, the processor 20 determines whether the variation characteristic of the oxygen concentration detection value meets a preset variation condition. The process proceeds to operation 213 when the variation characteristic of the oxygen concentration detection value meets the preset variation condition, and the process returns to operation 211 when the variation characteristic of the oxygen concentration detection value does not meet the preset variation condition.

[**0056]** In operation 213, the processor 20 determines the target time or the target time interval based on a time or time interval where the variation characteristic meets the preset variation condition. The interference of the interference gas with the oxygen concentration detection meeting the preset condition includes that the variation characteristic of the oxygen concentration detection value meets the preset variation condition.

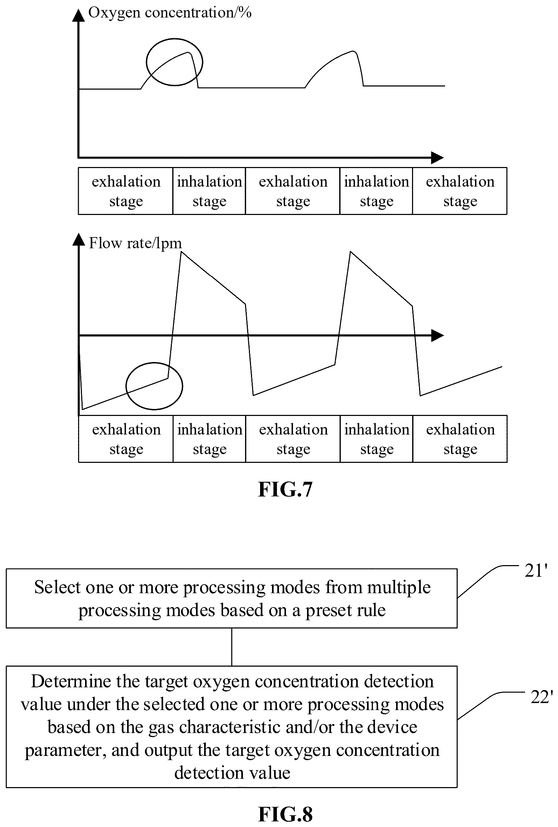

[**0057]** The influence of backflow of CO₂ on the oxygen concentration detection during ventilation is shown in FIG. 7. In an exhalation stage, CO₂ in the exhaled gas by the patient enters the respiratory support device with a reverse airflow, which affects the detection of oxygen concentration by the ultrasonic oxygen sensor 30 in the respiratory support device, resulting in a relatively high oxygen concentration. If the interference gas mainly consists of water vapor, when the interference gas enters the respiratory support device and affects the detection of oxygen concentration by the ultrasonic oxygen sensor 30, it may result in a relatively low oxygen concentration.

[**0058]** As shown in FIG. 7, the oxygen concentration detection value fluctuates greatly when the detection is affected by backflow of CO₂, while the oxygen concentration detection value is relatively stable when the detection is not affected by backflow of CO₂. The oxygen concentration detection value may be selectively read by identifying a stage where the oxygen concentration detection value is stable. That is, the preset variation condition is that the oxygen concentration detection value is stable (reflecting that the oxygen concentration is not affected by backflow of CO₂), such that the target time or the target time interval where the oxygen concentration detection value is stable may be determined, and thus the stable oxygen concentration detection value may be outputted in the operation 22.

[**0059]** The variation characteristic of the oxygen concentration detection value may reflect whether the oxygen concentration is stable or not, and specifically, the variation characteristic of the oxygen concentration detection value may include, but is not limited to a slope, a variance, etc.

[**0060]** For example, the variation characteristic of the oxygen concentration detection value includes a slope of the oxygen concentration detection value, and the preset variation condition corresponding thereto includes: a duration during which the slope of the oxygen concentration detection value does not exceed a preset slope reaching a preset first time length. As shown in FIG. 7, the greater the slope, the more unstable the oxygen concentration; when the slope does not exceed the preset slope in the first time length, it indicates that the oxygen concentration is very stable, that is, the ultrasonic oxygen sensor 30 is not interfered by the interference gas. In other words, if the duration during which the slope of the oxygen concentration detection value does not exceed the preset slope reaches the first time length, it indicates that the detection of oxygen concentration at such time or time interval is not interfered by the interference gas, and such time or time interval is used as the target time or the target time interval. Therefore, in the operation 22, the target oxygen concentration detection value may be obtained based on the oxygen concentration detection value at the target time or in the target time interval. It is apparent that the target oxygen concentration detection value eliminates the interference of the interference gas to the greatest extent, and improves detection accuracy. The preset slope may be set as required, for example, the preset slope may be set as 0 or a value close to 0. The first time length may also be set as required, for example, the first time length may be set as a value in a range of 20 ms to 1 s, and 300 ms is used as the first time length in the embodiment.

[0061] For another example, the variation characteristic of the oxygen concentration detection value includes a variance of the oxygen concentration detection value, and the preset variation condition corresponding thereto includes: the variance of the oxygen concentration detection value does not exceed a preset variance. The variance may also reflect stability of a group of oxygen concentration detection values. As an equivalent replacement of the variance, a standard deviation also falls within the scope of protection of the variance. When the variance of the oxygen concentration detection value does not exceed the preset variance, it indicates that the detection of oxygen concentration at such time or time interval is not interfered by the interference gas, and such time or time interval is used as the target time or the target time interval. Therefore, in the operation 22, the target oxygen concentration detection value may be obtained based on the oxygen concentration detection value at the target time or in the target time interval. Similarly, the target oxygen concentration detection value eliminates the interference of the interference gas to the greatest extent, and improves detection accuracy. The preset variance may be set as required.

[**0062]** In an embodiment where a second processing mode is used, the processor 20 determines the target oxygen concentration detection value under the second processing mode based on the gas characteristic and/or the device parameter, and outputs the target oxygen concentration detection value. For example, the device parameter includes a respiratory rate and a respiratory ratio set by the respiratory support device. The processor 20 may determine the target time or the target time interval based on the respiratory rate and the respiratory ratio set by the respiratory support device. Of course, the processor 20 may also determine the target time or the target time interval based on a respiratory rate and a respiratory ratio detected by the respiratory support device. After determining the target time or the target time interval, the processor 20 may determine the target oxygen concentration detection value based on the oxygen concentration detection value at the target time or in the target time interval, and output the target oxygen concentration detection value during at least part of the treatment time period.

[**0063]** As shown in FIG. 7, the ultrasonic oxygen sensor is not continuously interfered by the interference gas, instead, the ultrasonic oxygen sensor is periodically interfered by the interference gas with the patient's respiration. The oxygen concentration is very stable during a time interval before an end of inhalation, which indicates that the ultrasonic oxygen sensor is not interfered by the interference gas during the time interval. Therefore, the processor 20 may specifically determine a respiratory phase based on the respiratory rate and the respiratory ratio, that is, determine whether each time is in an inhalation stage or an exhalation stage. Furthermore, when the patient may breathe spontaneously without setting the respiratory rate and the respiratory ratio, a respiratory phase may be determined based on a waveform of the air characteristic. Specifically, such air characteristic may include at least one of: a flow rate detection value of a gas flowing through the ultrasonic oxygen sensor detected by the ultrasonic oxygen sensor, a flow rate detection value of a gas flowing through the ultrasonic oxygen sensor detected by a flow rate sensor, a flow rate detection value of the gas in the respiratory gas flow path detected by the flow rate sensor, or a pressure detection value of the gas in the respiratory gas flow path detected by a pressure sensor. When the patient breathes spontaneously, the flow rate and the pressure in the pipeline may change regularly, therefore it may be identified from their waveforms that inhalation occurs during which time interval and exhalation occurs during which time interval. In the embodiment, the processor 20 may determine the respiratory phase from any one of a waveform of the flow rate detection value and a waveform of the pressure detection value. Of course, the processor 20 may also determine the respiratory phase by using only one of the waveform of the flow rate detection value and the waveform of the pressure detection value; however, accuracy thereof is not as high as accuracy of determining the respiratory phase by using the two waveforms. When the respiratory phase is an inhalation stage of a respiratory cycle, the processor 20 sets a time within a time interval corresponding to a preset second time length before an end of inhalation, as the target time; or, when the respiratory phase is the inhalation stage of the respiratory cycle, the processor 20 sets the time interval corresponding to the preset second time length before the end of inhalation, as the target time interval. Since the oxygen concentration detection value at each time in the preset second time length before the end of inhalation is very stable, it is considered that the oxygen concentration detection value is not interfered by the interference gas, then the time interval corresponding to the preset second time length before the end of inhalation or a time within the time interval may be used as the target time interval or the target time. That is, the interference of the interference gas with the oxygen concentration detection meets the preset condition in the preset second time length before the end of inhalation. Therefore, in the operation 22, the target oxygen concentration detection value may be obtained based on the oxygen concentration detection value at the target time or in the target time interval. Similarly, the target oxygen concentration detection value eliminates the interference of the interference gas to the greatest extent, and improves detection accuracy. As shown in FIG. 7, in some embodiments, the detection is not interfered by the interference gas during a time interval in the exhalation stage; however, for the sake of safety, only a time or time interval in the inhalation stage may be selected as the target time or the target time interval, that is, the target time or the target time interval is within the inhalation stage, and it is difficult for the interference gas exhaled by the patient at such time or time interval to flow to a position where the ultrasonic oxygen sensor is located.

[**0064]** The second time length may be set as required, for example, the second time length may be set as a value in a range of 20 ms to 1 s, and an embodiment is described by an example of using 200 ms as the second time length.

[**0065]** In an embodiment where a third processing mode is used, the processor 20 determines the target oxygen concentration detection value under the third processing mode based on the gas characteristic and/or the device parameter, and outputs the target oxygen concentration detection value. For example, the gas characteristic includes a flow direction of the gas in the respiratory gas flow path 10 detected by a flow rate sensor 40. The processor 20 may determine the target time or the target time interval based on the flow direction of the gas in the respiratory gas flow path 10 detected by the flow rate sensor 40, determine the target oxygen concentration detection value based on the oxygen concentration detection value at the target time or in the target time interval, and output the target oxygen concentration detection value during at least part of the treatment time period.

[**0066]** As shown in FIG. 7, the value detected by the ultrasonic oxygen sensor is not affected by backflow of CO₂ during a time interval after backflow of CO₂ occurs, therefore the value detected by the ultrasonic oxygen sensor during the time interval may be read as the target oxygen concentration detection value. Specifically, when the flow direction of the gas in the respiratory gas flow path 10 detected by the flow rate sensor 40 changes from toward the patient to away from the patient, the processor 20 sets a time, at which time a preset third time length has elapsed from the time when the flow direction changes from toward the patient to away from the patient, as a start time; the processor 20 sets a time within a time interval, which time interval begins from the start time and lasts for a preset fourth time length, as the target time, or sets the time interval, which time interval begins from the start time and lasts for the preset fourth time length, as the target time interval. That is, after the gas in the respiratory gas flow path 10 flows in a direction away from the patient (backflow occurs, that is, the gas flows into the respiratory support device), a duration of the backflow is not too long, then as long as it waits for a longer time, that is, after the third time length has elapsed, it is considered that no backflow occurs in the respiratory gas flow path 10 and no interference gas passes through the ultrasonic oxygen sensor, therefore a time or a time interval after this time (the start time) may be used as the target time or the target time interval. That is, when the flow direction of the gas in the respiratory gas flow path 10 changes from toward the patient to away from the patient, the preset third time length has elapsed from the time when the flow direction changes from toward the patient to away from the patient, then during a subsequent time interval (the fourth time length), the interference of the interference gas with the oxygen concentration detection meets the preset condition (that is, the oxygen concentration detection is not interfered by the interference gas). Therefore, in the operation 22, the target oxygen concentration detection value may be obtained based on the oxygen concentration detection value at the target time or in the target time interval. Similarly, the target oxygen concentration detection value eliminates the interference of the interference gas to the greatest extent, and improves detection accuracy. The third time length may be set as required, for example, the third time length may be set as a value in a range of 300 ms to 1 s; similarly, the fourth time length may be set as required, for example, the fourth time length may be set as a value in a range of 0 to 200 ms, and an embodiment is described by an example of using 50 ms as the fourth time length.

[**0067]** In some embodiments, if the interference of the interference gas exists in each respiratory cycle, it is preferable that the time when the flow direction changes from toward the patient to away from the patient is in the same respiratory cycle as the target time or the target time interval, such that cross-respiratory cycle does not occur, and the oxygen concentration acquired during the interference is not used as the target oxygen concentration detection value, either.

[**0068]** In an embodiment where a fourth processing mode is used, the processor 20 determines the target oxygen concentration detection value under the fourth processing mode based on the gas characteristic and/or the device parameter, and outputs the target oxygen concentration detection value. For example, the gas characteristic includes a flow rate detection value of a gas flowing through the ultrasonic oxygen sensor 30 detected by the ultrasonic oxygen sensor 30 or a flow rate sensor 40. The processor 20 may determine the target time or the target time interval based on the flow rate detection value of the gas flowing through the ultrasonic oxygen sensor 30, determine the target oxygen concentration detection value based on the oxygen concentration detection value at the target time or in the target time interval, and output the target oxygen concentration detection value during at least part of the treatment time period.

[0069] As shown in FIG. 7, if the interference gas enters the respiratory gas flow path 10, the interference gas may be flushed away by ventilation of the patient in the inhalation stage, and the oxygen concentration acquired by the ultrasonic oxygen sensor 30 is accurate as long as it is determined that the interference gas has been flushed away. Therefore, the processor 20 may calculate, in an inhalation stage of a respiratory cycle, a volume of the gas flowing through the ultrasonic oxygen sensor 30 in the inhalation stage based on the flow rate detection value (for example, volume = flow rate detection value * time). The inhalation stage of the respiratory cycle is known to the respiratory support device, for example, the processor 20 may determine the respiratory phase based on the respiratory rate and the respiratory ratio, and thus may know which time interval is the inhalation stage. Of course, the device parameter acquired by the processor 20 may further include an output flow rate of the oxygen-containing gas, then the processor 20 calculates, in an inhalation stage of a respiratory cycle, a volume of the gas flowing through the ultrasonic oxygen sensor in the inhalation stage based on the output flow rate of the oxygen-containing gas. The volume calculated in this way is equivalent to a theoretical volume, and the volume calculated by the flow rate detection value as mentioned above is equivalent to an actual volume, both of which may be applicable. When the volume exceeds a preset volume, the processor 20 sets a time, at which time a preset fifth time length has elapsed from the time when the volume exceeds the preset volume, as a start time; the processor 20 sets a time within a time interval, which time interval begins from the start time and lasts for a preset sixth time length, as the target time, or sets the time interval, which time interval begins from the start time and lasts for the preset sixth time length, as the target time interval. The preset volume may be set as required, and usually, the preset volume may be a volume of a gas path where the ultrasonic oxygen sensor 30 is located. For example, the ultrasonic oxygen sensor 30 is arranged in the respiratory gas flow path 10, and the preset volume may be a volume of the respiratory gas flow path 10. For another example, as shown in FIG. 2 and FIG. 3, the respiratory gas flow path 10 is provided with a bypass, the ultrasonic oxygen sensor 30 is arranged in the bypass, and the preset volume may be a volume of the bypass. When the volume exceeds the preset volume, it theoretically indicates that an original gas in the gas path where the ultrasonic oxygen sensor 30 is located has been flushed away completely; however, for the sake of safety, it may wait for a certain time (the fifth time length) and subsequently, a time or time interval may be used as the target time or the target time interval, to ensure that the ultrasonic oxygen sensor 30 is not interfered by the interference gas at the target time or in the target time interval. That is, timing starts when the volume exceeds the preset volume, then in the time (the sixth time length) occurring after the preset fifth time length has elapsed, the interference of the interference gas with the oxygen concentration detection meets the preset condition. Therefore, in the operation 22, the processor 20 may obtain the target oxygen concentration detection value based on the oxygen concentration detection value at the target time or in the target time interval. Similarly, the target oxygen concentration detection value eliminates the interference of the interference gas to the greatest extent, and improves detection accuracy.

[**0070]** As a time margin, the fifth time length may be set as required, for example, the fifth time length may be set as a value in a range of 300 ms to 1 s; of course, the fifth time length may be 0, that is, there is no time margin. The sixth time length may also be set as required, for example, the sixth time length may be set as a value in a range of 0 to 200 ms. When the sixth time length is 0, the target time is a time when the fifth time length ends, and when the sixth time length is 200 ms, the target time may be a time in a range of 0 to 200 ms. The target time interval may be the entire sixth time length.

[0071] As shown in FIG. 2 and FIG. 3, an embodiment is described by an example of arranging the ultrasonic oxygen sensor 30 in the bypass. In the exhalation stage, the exhaled gas by the patient enters a branch where the ultrasonic oxygen sensor 30 is located, and a flow path of the exhaled gas is shown by arrows in FIG. 2. In the inhalation stage, a fresh gas enters the branch where the ultrasonic oxygen sensor 30 is located, and flushes away a residual gas from the exhalation stage, and a flow path of the fresh gas is shown by arrows in FIG. 3. Therefore, a volume of the gas flowing through the branch where the ultrasonic oxygen sensor 30 is located is calculated in the inhalation stage. When the volume of the gas exceeds a total volume of the bypass branch, it indicates that a residual interference gas such as CO₂ or the like has been flushed away completely. A detection value of the ultrasonic oxygen sensor read at this time is a value which is not interfered by CO₂.

[**0072]** In the foregoing embodiments, the respiratory support device determines the target oxygen concentration detection value with only one fixed processing mode (mainly by determining the target time or the target time interval). In several embodiments described below, the respiratory support device has multiple processing modes, and the processor 20 selects one or more processing modes from the multiple processing modes based on situations, to determine the target oxygen concentration detection value.

[**0073]** In an embodiment, the respiratory support device has multiple processing modes, and the above operation 2 may specifically include the following operations 21' and 22'.

[0074] In operation 21', the processor 20 selects one or more processing modes from multiple processing modes based on a preset rule. The multiple processing modes may include multiple ones of the first to fourth processing modes as described above. The embodiment is described by an example that the multiple processing modes include the above four processing modes.

[**0075]** Actually, the preset rule is a selection rule for the processing modes. For example, the preset rule may include a preset priority rule for the multiple processing modes, for example, if the oxygen concentration obtained under one of the processing modes is inaccurate, other processing modes are used; the preset rule may further include and/or an accuracy judgment rule for the multiple processing modes, for example, the detection of oxygen concentration is made once under all of the multiple processing modes, and a most accurate oxygen concentration is selected from oxygen concentrations obtained under the multiple processing modes. That is, the target oxygen concentration detection value obtained under the processing mode with a high priority and/or a high accuracy may be selected and outputted.

[**0076]** In operation 22', the processor 20 determines the target oxygen concentration detection value under the selected one or more processing modes based on the gas characteristic and/or the device parameter, and outputs the target oxygen concentration detection value. Each of the foregoing processing modes has its advantages and disadvantages, and the target oxygen concentration detection value obtained under the processing mode selected after making priority and/or accuracy judgment will be more accurate.

[**0077]** Based on different preset rules, there are multiple specific implementations for the operation 21' and the operation 22', and some of them will be described below respectively.

[**0078]** In a first implementation, the gas characteristic includes a flow direction of the gas in the respiratory gas flow path 10 detected by a flow rate sensor. In the operation 2, after the flow direction changes from toward the patient to away from the patient, the processor 20 may select a first processing mode, determine the target oxygen concentration detection value under the first processing mode based on the gas characteristic and/or the device parameter, and output the target oxygen concentration detection value. There is a large possibility that gases from the outside flowing into the respiratory gas flow path 10 cause interference with the oxygen concentration detection, that is, they may cause fluctuation of the oxygen concentration value, therefore it is more appropriate to use the first processing mode, and specific processes of determining the target oxygen concentration detection value under the first processing mode have been described in detail in the foregoing embodiments and will not be elaborated here.

[**0079]** In a second implementation, the processor 20 selects a first processing mode first, and if the target oxygen concentration detection value may be determined under the first processing mode based on the gas characteristic and/or the device parameter, the processor 20 outputs the determined target oxygen concentration detection value. A specific implementation thereof may refer to the foregoing embodiments, the foregoing first implementation or the like, and will not be elaborated here. That is, the target oxygen concentration detection value is determined preferentially under the first processing mode; however, the target oxygen concentration detection value may not be necessarily obtained under the first processing mode, for example, the target time and the target time interval are unable to be determined since the oxygen concentration is unstable all the way due to flow rate control, and thus the target oxygen concentration detection value is unable to be determined; in this situation, the target oxygen concentration detection value may be determined under other processing modes. That is, when the target oxygen concentration detection value is not capable of being determined under the first processing mode, the processor 20 selects a second processing mode, a third processing mode and/or a fourth processing mode, and if the target oxygen concentration detection value may be determined under the selected processing mode based on the gas characteristic and/or the device parameter, the processor 20 outputs the determined target oxygen concentration detection value. The first processing mode, the second processing mode, the third processing mode and the fourth processing mode are different from one another. In this way, it may avoid a situation where the target oxygen concentration detection value is unable to be determined.

[**0080]** The device parameter includes a respiratory ratio set by the respiratory support device. The processor 20 may specifically select the second processing mode, the third processing mode and/or the fourth processing mode as follows. When the respiratory ratio is greater than a preset ratio, the processor 20 may determine the target oxygen concentration detection value under the second processing mode and/or the fourth processing mode, and if the target oxygen concentration detection value may be determined under the second processing mode and/or the fourth processing mode, the processor 20 may output the target oxygen concentration detection value. When the respiratory ratio is relatively large, the exhalation stage occupies a relatively large proportion, and there is a greater amount of interference gas entering into the respiratory gas flow path. It may be known from principles of the second processing mode and the fourth processing mode that, it is more accurate to determine the target time or the target time interval that is not interfered by the interference gas, under at least one of the two processing modes. Correspondingly, when the respiratory ratio is less than the preset ratio, the processor 20 may determine the target oxygen concentration detection value under the third processing mode, and if the target oxygen concentration detection value may be determined under the third processing mode, the processor 20 may output the target oxygen concentration detection value. It is an empirical calculation to determine the target time and the target time interval under the third processing mode, and it is simple and reliable to use the third processing mode when the exhalation stage occupies a relatively small proportion.

[**0081]** Of course, it may also replace the respiratory ratio with a time length of the exhalation stage or a time length of the inhalation stage, such that the respiratory support device may be used when the patient breathes spontaneously without setting the respiratory ratio. Specifically, the gas characteristic acquired by the processor 20 may include at least one of: a flow rate detection value of a gas flowing through the ultrasonic oxygen sensor detected by the ultrasonic oxygen sensor, a flow rate detection value of a gas flowing through the ultrasonic oxygen sensor detected by a flow rate sensor, a flow rate detection value of the gas in the respiratory gas flow path detected by the flow rate sensor, or a pressure detection value of the gas in the respiratory gas flow path detected by a pressure sensor. The processor 20 determines a time length of an exhalation stage in a respiratory cycle or a time length of an inhalation stage in a respiratory cycle based on a waveform of the gas characteristic, which is not essentially different from determination of the respiratory phase as mentioned above. When the time length of the exhalation stage is greater than a preset first threshold or the time length of the inhalation stage is less than a preset second threshold, if the target oxygen concentration detection value may be determined under the second processing mode and/or the fourth processing mode, the processor 20 outputs the target oxygen concentration detection value; when the time length of the exhalation stage is less than the preset first threshold or the time length of the inhalation stage is greater than the preset second threshold, if the target oxygen concentration detection value may be determined under the third processing mode, the processor 20 outputs the target oxygen concentration detection value. The first threshold and the second threshold may be set as required.

[**0082]** In short, no matter judgment or selection is made by using the respiratory ratio, the time length of the exhalation stage or the time length of the inhalation stage, the second processing mode and/or the fourth processing mode are selected when the exhalation stage is long, and the third processing mode is selected when the exhalation stage is short.

[**0083]** In a third implementation, the processor 20 determines target oxygen concentration detection values under multiple processing modes, then selects a target oxygen concentration detection value from the target oxygen concentration detection values and outputs the target oxygen concentration detection value. Specifically, the processor 20 determines the target oxygen concentration detection value under several of the first processing mode, the second processing mode, the third processing mode and the fourth processing mode based on the gas characteristic and/or the device parameter. The target oxygen concentration detection values determined under the several processing modes respectively are referred to as candidate target oxygen concentration detection values, to facilitate distinguishing them from the target oxygen concentration detection value outputted finally, and specific processes of determining the target oxygen concentration detection values (candidate target oxygen concentration detection values) under the several processing modes may refer to the foregoing embodiments, and will not be elaborated here. Multiple candidate target oxygen concentration detection values may be determined under the several processing modes, and the processor 20 may select, based on a type of the interference gas, a corresponding candidate target oxygen concentration detection value from the multiple candidate target oxygen concentration detection values as the target oxygen concentration detection value, and output the target oxygen concentration detection value. Different types of interference gases have different influences on the oxygen concentration. Therefore, the candidate target oxygen concentration detection values may be screened based on the type of the interference gas, such that the target oxygen concentration detection value outputted finally is more accurate.

[**0084]** Specifically, when the type of the interference gas is carbon dioxide (CO₂), the processor 20 may select a minimum candidate target oxygen concentration detection value from the multiple candidate target oxygen concentration detection values as the target oxygen concentration detection value, and output the target oxygen concentration detection value. CO₂ is heavier than oxygen, and the ultrasonic oxygen sensor 30 may treat CO₂ as oxygen to result in a relatively high oxygen concentration, therefore it is more appropriate to select and output the minimum candidate target oxygen concentration detection value. When the type of the interference gas is water vapor, the processor 20 may select a maximum candidate target oxygen concentration detection value from the multiple candidate target oxygen concentration detection values as the target oxygen concentration detection value, and output the target oxygen concentration detection value. Water vapor is lighter than oxygen, and the ultrasonic oxygen sensor 30 may treat water vapor as oxygen to result in a relatively low oxygen concentration, therefore it is more appropriate to select and output the maximum candidate target oxygen concentration detection value.

[**0085]** It may be seen that each of the above three implementations may adaptively select an appropriate processing mode to determine the time or time interval that is not interfered by the interference gas, thereby determining and outputting an accurate oxygen concentration detection value, further improving the accuracy of oxygen concentration detection.

[**0086]** In the foregoing various embodiments, after determining the target time or the target time interval that is not interfered by the interference gas, the target oxygen concentration detection value is determined based on the oxygen concentration detection value at the target time or in the target time interval, and the target oxygen concentration detection value is outputted (for example, during at least part of the treatment time period), of which multiple specific implementations may be present. Some of them will be given and described below.

[**0087]** In a first method, the processor 20 sets the oxygen concentration detection value at the target time as the target oxygen concentration detection value, and outputs the target oxygen concentration detection value. The oxygen concentration detection value at the target time is not interfered by the interference gas, and thus may be directly displayed as the oxygen concentration.

[**0088]** In a second method, the processor 20 calculates an average value of the oxygen concentration detection values at multiple times in the target time interval, sets the average value as the target oxygen concentration detection value, and outputs the target oxygen concentration detection value. All of the oxygen concentration detection values at the target time interval are not interfered by the interference gas, therefore an average value of multiple oxygen concentration detection values acquired by the ultrasonic oxygen sensor during the target time interval may be taken, and the average value may be displayed as the oxygen concentration.

[**0089]** In a third method, when multiple target times or multiple target time intervals exist within a same respiratory cycle, the processor selects, based on a preset selection rule, one of the oxygen concentration detection values at the multiple target times or in the multiple target time intervals within the same respiratory cycle as the target oxygen concentration detection value, and outputs the target oxygen concentration detection value. For example, the processor selects a minimum or maximum one of the oxygen concentration detection values at the multiple target times or in the multiple target time intervals within the same respiratory cycle as the target oxygen concentration detection value, and outputs the target oxygen concentration detection value. Specifically, whether the minimum or maximum oxygen concentration detection value is selected, may be determined based on the type of the interference gas. For example, when the type of the interference gas is carbon dioxide, the minimum oxygen concentration detection value is selected as the target oxygen concentration detection value; and when the type of the interference gas is water vapor, the maximum oxygen concentration detection value is selected as the target oxygen concentration detection value.

**[0090]** In a fourth method, when multiple target times or multiple target time intervals exist within a same respiratory cycle, the processor 20 determines the target oxygen concentration detection value based on the oxygen concentration detection values at the multiple target times or in the multiple target time intervals within the same respiratory cycle, and outputs the target oxygen concentration detection value. For example, the processor 20 calculates an average value of the oxygen concentration detection values at the multiple target times or in the multiple target time intervals within the same respiratory cycle, sets the average value as the target oxygen concentration detection value, and outputs the target oxygen concentration detection value.

[**0091]** The target oxygen concentration detection value may be outputted directly. Of course, it may also determine whether the target oxygen concentration detection value is within a normal range. Only the target oxygen concentration detection value within the normal range is outputted, to avoid outputting an invalid target oxygen concentration detection value. Specifically, the oxygen concentration in the gas outputted by the respiratory support device to the patient usually does not exceed a normal value range (such as 21% to 100%). If the target oxygen concentration detection value exceeds the normal value range, it indicates that the detection value is meaningless and may be caused by other reasons (a non-interference gas). Therefore, the processor 20 may determine whether the target oxygen concentration detection value is within a preset normal value range; if the target oxygen concentration detection value is within the preset normal value range, the processor 20 may output the target oxygen concentration detection value; and if the target oxygen concentration detection value is not within the preset normal value range, the processor 20 may determine, based on the oxygen concentration detection value during the treatment time period, an oxygen concentration detection value required to be outputted, and output the detection value. When the target oxygen concentration detection value is not within the normal value range, an oxygen concentration detection value required to be outputted is determined based on the oxygen concentration detection value during the treatment time period, which may specifically be that oxygen concentration detection values that are not within the normal value range may be deleted, and an average value of remaining oxygen concentration detection values (that is, oxygen concentration detection values within the normal value range) may be taken, and the average value may be outputted as the oxygen concentration.

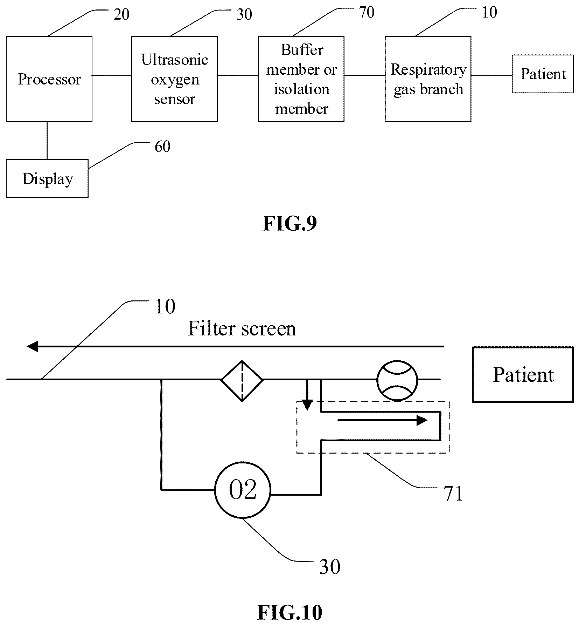

[**0092]** In the above embodiments, the oxygen concentration detection values acquired by the ultrasonic oxygen sensor are screened mainly, and the detection value that is not interfered is selected and outputted. In fact, it may also avoid the interference gas from coming into contact with the ultrasonic oxygen sensor, by improve hardware structures of the respiratory support device, for example, improving the gas path, which may also improve the accuracy of oxygen concentration detection. Specifically, as shown in FIG. 9, such a respiratory support device includes a respiratory gas flow path 10, an ultrasonic oxygen sensor 30, a buffer member 70 or an isolation member 70, and a processor 20.

[**0093]** The respiratory gas flow path 10 is configured to connect to a patient and deliver an oxygen-containing gas to the patient to provide respiratory support. The respiratory gas flow path 10 further optionally contains an interference gas.

[**0094]** The ultrasonic oxygen sensor 30 is configured to detect an oxygen concentration of a gas in the respiratory gas flow path 10. The gas in the respiratory gas flow path 10 includes at least the oxygen-containing gas and optionally the interference gas.

[**0095]** That is, the respiratory gas flow path 10 in the embodiment has the same function, structure or the like as those of the respiratory gas flow path in the above embodiments, and functions of the ultrasonic oxygen sensor 30 are also the same as those of the ultrasonic oxygen sensor in the above embodiments, and will not be elaborated here.

[**0096]** The buffer member 70 or the isolation member 70 is arranged at a front end or a rear end of the ultrasonic oxygen sensor 30, an end of the ultrasonic oxygen sensor 30 close to the patient is the rear end thereof, and the buffer member 70 or the isolation member 70 shown in FIG. 9 is arranged at the rear end of the ultrasonic oxygen sensor 30. The buffer member 70 or the isolation member 70 is configured to reduce or block the interference gas from entering into a detection range of the ultrasonic oxygen sensor 30. The buffer member 70 or the isolation member 70 may isolate the interference gas physically, thereby improving the accuracy of oxygen concentration detection.

[**0097]** The processor 20 is configured to acquire an oxygen concentration detection value of the gas in the respiratory gas flow path 10 detected by the ultrasonic oxygen sensor 30, determine a target oxygen concentration detection value based on the oxygen concentration detection value, and output the target oxygen concentration detection value. Specifically, the processor 20 may directly set the oxygen concentration detection value as the target oxygen concentration detection value, and output the target oxygen concentration detection value; or, the processor 20 may take an average value of oxygen concentration detection values (for example, an average value of oxygen concentration detection values in a respiratory cycle), set the average value as the target oxygen concentration detection value, and output the target oxygen concentration detection value.

[**0098]** The respiratory support device may further include a display 60. The processor 20 outputs the target oxygen concentration detection value, and may output the target oxygen concentration detection value to the display 60 to display it. In terms of hardware structures, the embodiment is equivalent to adding the buffer member or the isolation member, and other hardware is the same as those in the foregoing embodiments, and will not be elaborated here.

[**0099]** As shown in FIG. 10, the respiratory support device specifically includes a buffer member 70. The buffer member 70 includes a buffer gas path 71, and the buffer gas path 71 is arranged at the rear end of the ultrasonic oxygen sensor 30 and is configured to accommodate the interference gas through its own volume, such that the interference gas is unable to diffuse from the rear end of the ultrasonic oxygen sensor 30 into the ultrasonic oxygen sensor. The buffer gas path 71 is equivalent to a container capable of accommodating backflow of the interference gas. In the exhalation stage, backflow of CO₂ gas enters the buffer gas path 71 while does not reach inside of the ultrasonic oxygen sensor 30, thereby isolating the interference gas and avoiding backflow of CO₂ from affecting monitoring of the ultrasonic oxygen sensor 30. Each time the patient breathes, the interference gas has a limited volume, which usually does not exceed a value. If the buffer gas path has a volume no less than the value, the interference gas may be isolated well. For example, the buffer gas path 71 may include a pipeline with a volume no less than a preset value, or the buffer gas path 71 may include an air container with a volume no less than the preset value. The preset value may be a value between 20-40 milliliters, and in the embodiment, the preset value is 30 milliliters.

[**0100]** Arrangement of the buffer gas path 71 on the gas path where the ultrasonic oxygen sensor 30 is located may effectively reduce the interference of the interference gas to the ultrasonic oxygen sensor 30. In the embodiment, groups of comparative experiments are made using the respiratory support device, which will be described in detail below.

[**0101]** Experimental conditions are as follows. The respiratory support devices operates in a Volume Assured Pressure Support (VAPS) mode, a tidal volume is 1500 mL, an exhalation pressure is 5cmH2O, an inhalation time 2s, a respiratory rate is 10/min. The VAPS mode is an abbreviation of Volume Assured Pressure Support mode, it is an intelligent ventilation mode combining characteristics of Pressure Support Ventilation (PSV) and Volume Controlled Ventilation (VCV).

[**0102]** Variables for the groups of experiments (that is, difference between the groups of experiments) are: whether there is a buffer gas path 71 and whether backflow of CO₂ is provided from the patient side.

[**0103]** Variable conditions for an experimental group A are: there is no buffer gas path 71 in the respiratory support device, and backflow of CO₂ is not provided (no backflow of CO₂ is) from the patient side (that is, there is no interference of the interference gas). The respiratory support device outputs oxygen-containing gases with oxygen concentrations of 21%, 30% and 60% respectively. Each time the respiratory support device outputs an oxygen-containing gas with a certain oxygen concentration, a CO₂ concentration is measured at the exhalation valve of the respiratory support device by using a sensor, to obtain a CO₂ monitoring value at the patient side; an oxygen analyzer is used as a standard detection device for oxygen concentration, to measure the oxygen concentration of the gas in the respiratory gas flow path, to obtain an oxygen analyzer-measured value; the ultrasonic oxygen sensor 30 measures the oxygen concentration of the gas in the respiratory gas flow path (it should be noted that the foregoing method for detecting an oxygen concentration is not used; instead, the ultrasonic oxygen sensor 30 directly detects the oxygen concentration), and obtains an ultrasonic oxygen measurement value. These measured data are summarized as shown in Table A below.

**Table A**

| Oxygen concentration set value (vol.%) | CO₂ monitoring value at the patient side | Oxygen analyzer-measured value (vol.%) | Ultrasonic oxygen measurement value (vol.%) | Error (vol.%) |
|---|---|---|---|---|
| 21 | 0 | 21.02 | 22 | -0.98 |
| 30 | 0 | 30.02 | 30 | 0.02 |
| 60 | 0 | 61.86 | 61 | 0.86 |

[**0104]** Since backflow of CO₂ is not provided from the patient side in the experimental group A, all CO₂ monitoring values at the patient side are 0. As may be known from the above Table A, when it is known that the respiratory support device provides three different oxygen concentrations (21%, 30% and 60%) including a low oxygen concentration, a moderate oxygen concentration and a high oxygen concentration, the oxygen concentration of the gas in the respiratory gas flow path measured by the standard detection device is 21.02%, 30.02% and 61.86% respectively, while the oxygen concentration measured by the ultrasonic oxygen sensor 30 under the same condition is 22%, 30% and 61% respectively, and it is known by calculation that the measurement error of the ultrasonic oxygen sensor 30 is -0. 98%, 0.02%, and 0.86% respectively. As may be seen, the measurement error of the ultrasonic oxygen sensor 30 is ≤ 1%, in case that there is no interference of the interference gas and no buffer gas path 71 is provided.

[**0105]** Variable conditions for an experimental group B are: there is no buffer gas path 71 in the respiratory support device, backflow of CO₂ is provided from the patient side (a device that simulates exhalation of the gas by the patient and may output CO₂) (that is, there is the interference of the interference gas), and a CO₂ concentration in the backflow of CO₂ provided from the patient side is about 5%. The respiratory support device outputs oxygen-containing gases with oxygen concentrations of 21%, 30% and 60% respectively. Each time the respiratory support device outputs an oxygen-containing gas with a certain oxygen concentration, a CO₂ concentration is measured at the exhalation valve of the respiratory support device by using a sensor, to obtain a CO₂ monitoring value at the patient side; an oxygen analyzer is used as a standard detection device for oxygen concentration, to measure the oxygen concentration of the gas in the respiratory gas flow path, to obtain an oxygen analyzer-measured value; the ultrasonic oxygen sensor 30 measures the oxygen concentration of the gas in the respiratory gas flow path (it should be noted that the foregoing method for detecting an oxygen concentration is not used; instead, the ultrasonic oxygen sensor 30 directly detects the oxygen concentration), and obtains an ultrasonic oxygen measurement value. These measured data are summarized as shown in Table B below.

**Table B**

| Oxygen concentration set value (vol.%) | CO₂ monitoring value at the patient side | Oxygen analyzer-measured value (vol.%) | Ultrasonic oxygen measurement value (vol.%) | Error (vol.%) |
|---|---|---|---|---|
| 21 | 4.5 | 20.71 | 29 | -8.29 |
| 30 | 4.5 | 31.57 | 40 | -8.43 |
| 60 | 4.2 | 67.29 | 76 | -8.71 |

[**0106]** As may be known from the above Table B, when it is known that the respiratory support device provides three different oxygen concentrations (21%, 30% and 60%) including a low oxygen concentration, a moderate oxygen concentration and a high oxygen concentration, the oxygen concentration of the gas in the respiratory gas flow path measured by the standard detection device is 20.71%, 31.57% and 67.29% respectively, while the oxygen concentration measured by the ultrasonic oxygen sensor 30 under the same condition is 29%, 40% and 76% respectively, and it is known by calculation that the measurement error of the ultrasonic oxygen sensor 30 is -8.29%, -8.43%, and -8.71% respectively. As may be seen, the measurement error of the ultrasonic oxygen sensor 30 is about 8%, in case that there is interference of CO₂ with a concentration of about 5%, no buffer gas path 71 is provided, and the method for detecting an oxygen concentration according to the application is not used, that is, in case that there is the interference of the interference gas as in the related art. This error is large, which also indicates that the interference gas greatly affects the oxygen concentration detection of the ultrasonic oxygen sensor 30.

[**0107]** Variable conditions for an experimental group C are: the buffer gas path 71 is arranged in the respiratory support device, and backflow of CO₂ is not provided (no backflow of CO₂ is) from the patient side (that is, there is no interference of the interference gas). The respiratory support device outputs oxygen-containing gases with oxygen concentrations of 21%, 30% and 60% respectively. Each time the respiratory support device outputs an oxygen-containing gas with a certain oxygen concentration, a CO₂ concentration is measured at the exhalation valve of the respiratory support device by using a sensor, to obtain a CO₂ monitoring value at the patient side; an oxygen analyzer is used as a standard detection device for oxygen concentration, to measure the oxygen concentration of the gas in the respiratory gas flow path, to obtain an oxygen analyzer-measured value; the ultrasonic oxygen sensor 30 measures the oxygen concentration of the gas in the respiratory gas flow path (it should be noted that the foregoing method for detecting an oxygen concentration is not used; instead, the ultrasonic oxygen sensor 30 directly detects the oxygen concentration), and obtains an ultrasonic oxygen measurement value. These measured data are summarized as shown in Table C below.

**Table C**

| Oxygen concentration set value (vol.%) | CO₂ monitoring value at the patient side | Oxygen analyzer-measured value (vol.%) | Ultrasonic oxygen measurement value (vol.%) | Error (vol.%) |
|---|---|---|---|---|
| 21 | 0 | 21.07 | 23 | -1.93 |
| 30 | 0 | 27.59 | 30 | -2.41 |
| 60 | 0 | 58.81 | 60 | -1.19 |

[**0108]** Since backflow of CO₂ is not provided from the patient side in the experimental group C, all CO₂ monitoring values at the patient side are 0. As may be known from the above Table C, when it is known that the respiratory support device provides three different oxygen concentrations (21%, 30% and 60%) including a low oxygen concentration, a moderate oxygen concentration and a high oxygen concentration, the oxygen concentration of the gas in the respiratory gas flow path measured by the standard detection device is 21.07%, 27.59% and 58.81% respectively, while the oxygen concentration measured by the ultrasonic oxygen sensor 30 under the same condition is 23%, 30% and 60% respectively, and it is known by calculation that the measurement error of the ultrasonic oxygen sensor 30 is -1.93%, -2.41%, and -1.19% respectively. As may be seen, the measurement error of the ultrasonic oxygen sensor 30 is ≤ 3%, in case that there is no interference of the interference gas and the buffer gas path 71 is provided. This error is acceptable.

[**0109]** Variable conditions for an experimental group D are: the buffer gas path 71 is arranged in the respiratory support device, backflow of CO₂ is provided from the patient side (a device that simulates exhalation of the gas by the patient and may output CO₂) (that is, there is the interference of the interference gas), and a CO₂ concentration in the backflow of CO₂ provided from the patient side is about 5%. The respiratory support device outputs oxygen-containing gases with oxygen concentrations of 21%, 30% and 60% respectively. Each time the respiratory support device outputs an oxygen-containing gas with a certain oxygen concentration, a CO₂ concentration is measured at the exhalation valve of the respiratory support device by using a sensor, to obtain a CO₂ monitoring value at the patient side; an oxygen analyzer is used as a standard detection device for oxygen concentration, to measure the oxygen concentration of the gas in the respiratory gas flow path, to obtain an oxygen analyzer-measured value; the ultrasonic oxygen sensor 30 measures the oxygen concentration of the gas in the respiratory gas flow path (it should be noted that the foregoing method for detecting an oxygen concentration is not used; instead, the ultrasonic oxygen sensor 30 directly detects the oxygen concentration), and obtains an ultrasonic oxygen measurement value. These measured data are summarized as shown in Table D below.

**Table D**

| Oxygen concentration set value (vol.%) | CO₂ monitoring value at the patient side | Oxygen analyzer-measured value (vol.%) | Ultrasonic oxygen measurement value (vol.%) | Error (vol.%) |
|---|---|---|---|---|
| 21 | 5.2 | 20.63 | 24 | -3.37 |
| 30 | 5.2 | 26 | 30 | -4 |
| 60 | 4.8 | 61.11 | 63 | -1.89 |

[**0110]** As may be known from the above Table D, when it is known that the respiratory support device provides three different oxygen concentrations (21%, 30% and 60%) including a low oxygen concentration, a moderate oxygen concentration and a high oxygen concentration, the oxygen concentration of the gas in the respiratory gas flow path measured by the standard detection device is 20.63%, 26% and 61.11% respectively, while the oxygen concentration measured by the ultrasonic oxygen sensor 30 under the same condition is 24%, 30% and 63% respectively, and it is known by calculation that the measurement error of the ultrasonic oxygen sensor 30 is -3.37%, -4%, and -1.89% respectively. As may be seen, the measurement error of the ultrasonic oxygen sensor 30 is ≤ 4%, in case that there is interference of CO₂ with a concentration of about 5%, the buffer gas path 71 is provided, and the method for detecting an oxygen concentration according to the application is not used, that is, in the embodiment where the accuracy of the oxygen concentration detection value is improved by using the buffer gas path 71. It may be seen that compared to the related art represented by the experimental group B, the respiratory support device using the buffer gas path 71 according to the application effectively reduces the interference of the interference gas with the oxygen concentration detection (the maximum measurement error of 8.71% is reduced to a maximum of 4%).

[0111] Furthermore, the detection value of the ultrasonic oxygen sensor 30 when it is not interfered by the interference gas is used as the oxygen concentration value in the foregoing embodiments of the method for detecting an oxygen concentration, which is similar to the situation of the experimental group A, where there is no buffer gas path 71, and then the oxygen concentration which is not interfered by CO₂ is acquired, therefore it may be indirectly inferred that the measurement error of the ultrasonic oxygen sensor 30 in the foregoing method for detecting an oxygen concentration is ≤ 1%, and the foregoing embodiments of the method for detecting an oxygen concentration also greatly reduces the interference of the interference gas with the oxygen concentration detection compared to the measurement error exceeding 8% in the experimental group B (corresponding to the situation of the related art).

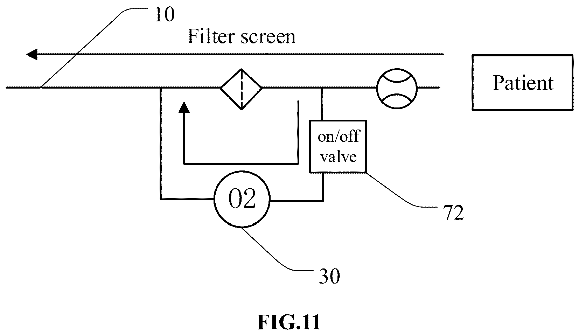

[**0112]** As shown in FIG. 11, the respiratory support device specifically includes an isolation member 70. The isolation member 70 includes an on/off valve 72, the respiratory gas flow path 10 is provided with a bypass, and both the on/off valve 72 and the ultrasonic oxygen sensor 30 are arranged in the bypass. The on/off valve 72 may be controlled by the processor 20 to be opened and closed, for example, the on/off valve is opened in an inhalation stage of a respiratory cycle and is closed in an exhalation stage of the respiratory cycle, such that the interference gas is unable to diffuse from the rear end of the ultrasonic oxygen sensor 30 into the ultrasonic oxygen sensor 30. The interference gas may also be isolated in this way.

[**0113]** It may be known from the above embodiments that the respiratory support device provided in the application effectively solves the problem of oxygen concentration detection interfered by the interference gas, such that the ultrasonic oxygen sensor may be applied to the respiratory support device. The ultrasonic oxygen sensor is a non-consumable with a long life (10 years), is resistant to vibration, and is more competitive than other sensors.

[**0114]** The application has been described with reference to various exemplary embodiments. However, it will be recognized by those skilled in the art that variation and modification may be made to the exemplary embodiments without departing from the scope of the application. For example, various operational steps as well as components for performing the operational steps, may be implemented in different modes according to a specific application or by considering any number of cost functions associated with operations of the system (for example, one or more steps may be deleted, modified or incorporated into other steps).

[**0115]** Furthermore, as understood by those skilled in the art, principle of the application may be reflected in a computer program product on a computer-readable storage medium, the computer-readable storage medium is pre-loaded with computer-readable program codes. Any tangible, non-transitory computer-readable storage medium may be used, including a magnetic storage device (a hard disk, a floppy disk, etc.), an optical storage device (a Compact Disk Read Only Memory (CD-ROM), a Digital Versatile Disk (DVD), a Blu Ray disk, etc.), a flash memory, etc. These computer program instructions may be loaded onto a general-purpose computer, a special-purpose computer or other programmable data processing devices to form a machine, such that these instructions executed on the computer or other programmable data processing devices may generate an apparatus for implementing a specified function. These computer program instructions may also be stored in a computer-readable memory, the computer-readable memory may instruct the computer or other programmable data processing devices to operate in a specific mode, such that the instructions stored in the computer-readable memory may form an article of manufacture which includes an implementation apparatus for implementing the specified function. The computer program instructions may also be loaded onto the computer or other programmable data processing devices, to perform a series of operational steps on the computer or other programmable devices to produce a computer-implemented process, such that the instructions executed on the computer or other programmable devices may provide steps for implementing the specified function.

[**0116]** While the principle of the application has been illustrated in various embodiments, many modifications of structures, arrangements, proportions, elements, materials and components that are specifically suitable for specific environmental and operational requirements may be used without departing from the principle and scope of the application. The above modifications and other variations or amendments will be included within the scope of the application.

[**0117]** The foregoing specific descriptions have been described with reference to various embodiments. However, it will be recognized by those skilled in the art that various modifications and variations may be made without departing from the scope of the application. Therefore, consideration of the application will be made in an illustrative sense rather than a restrictive sense, and all of the modifications will be included within the scope of the application. Furthermore, advantages relevant to various embodiments, other advantages, and solutions to problems have been described as above. However, benefits, advantages, solutions to problems, any element that may produce these benefits and advantages, or any solution that may make these benefits and advantages more explicit, should not be construed as being critical, necessary or essential. A term "include" and any other variants thereof as used here belongs to a non-exclusive inclusion, such that a process, method, article or device that includes a list of elements includes not only these elements, but also other elements that are not listed explicitly or that do not belong to the process, method, system, article or device. Furthermore, a term "couple" and any other variants thereof as used here refers to a physical connection, an electrical connection, a magnetic connection, an optical connection, a communication connection, a functional connection and/or any other connections.

[**0118]** It will be recognized by those skilled in the art that many variations may be made to details of the above embodiments without departing from basic principle of the application. Therefore, the scope of the application should be determined based on the following claims.

## Claims

1. A respiratory support device, comprising:
a respiratory gas flow path, configured to connect to a patient and deliver an oxygen-containing gas to the patient to provide respiratory support, the respiratory gas flow path further optionally containing an interference gas;
an ultrasonic oxygen sensor, configured to detect an oxygen concentration of a gas in the respiratory gas flow path, wherein the gas in the respiratory gas flow path comprises at least the oxygen-containing gas and optionally the interference gas;
a buffer member or an isolation member, arranged at a front end or a rear end of the ultrasonic oxygen sensor, and configured to reduce or block the interference gas from entering into a detection range of the ultrasonic oxygen sensor; and
a processor, configured to:
acquire a gas characteristic of the gas in the respiratory gas flow path and/or a device parameter of the respiratory support device; and
determine a target oxygen concentration detection value based on the gas characteristic and/or the device parameter, and optionally output the target oxygen concentration detection value.

2. The respiratory support device of claim 1, wherein
the respiratory gas flow path is configured to deliver the oxygen-containing gas to the patient during a treatment time period to provide the respiratory support, and the interference gas comprises at least part of an exhaled gas by the patient; and
the target oxygen concentration detection value is obtained based on an oxygen concentration detection value of the ultrasonic oxygen sensor at a target time or in a target time interval where an interference of the interference gas with the oxygen concentration detection meets a preset condition.

3. The respiratory support device of claim 2, further comprising a flow rate sensor and/or a pressure sensor, wherein the gas characteristic is obtained by the flow rate sensor, the pressure sensor and/or the ultrasonic oxygen sensor, wherein
the gas characteristic comprises one or more of: an oxygen concentration detection value of the gas in the respiratory gas flow path detected by the ultrasonic oxygen sensor, a flow rate detection value of a gas flowing through the ultrasonic oxygen sensor detected by the ultrasonic oxygen sensor, a flow direction of the gas in the respiratory gas flow path detected by the flow rate sensor, a flow rate detection value of a gas flowing through the ultrasonic oxygen sensor detected by the flow rate sensor, a flow rate detection value of the gas in the respiratory gas flow path detected by the flow rate sensor, and a pressure detection value of the gas in the respiratory gas flow path detected by the pressure sensor,
the device parameter comprises one or more of: a respiratory rate, a respiratory ratio and an output flow rate of the oxygen-containing gas set by the respiratory support device.

4. The respiratory support device of claim 2, wherein when determining the target oxygen concentration detection value based on the gas characteristic and/or the device parameter, and optionally outputting the target oxygen concentration detection value, the processor is further configured to:
determine the target time or the target time interval, determine the target oxygen concentration detection value based on the oxygen concentration detection value at the target time or in the target time interval, and output the target oxygen concentration detection value during at least part of the treatment time period,
wherein the at least part of the treatment time period comprises the target time or at least part of the target time interval, and other time(s) or at least part(s) of other time interval(s) than the target time or the target time interval.

5. The respiratory support device of claim 2, wherein the processor is further configured to:
correct an oxygen concentration detection value of the ultrasonic oxygen sensor at other time(s) or in at least part(s) of other time interval(s) than the target time or the target time interval, based on the gas characteristic and/or the device parameter, and output a corrected oxygen concentration detection value.

6. The respiratory support device of claim 4, wherein
the gas characteristic comprises an oxygen concentration detection value of the gas in the respiratory gas flow path detected by the ultrasonic oxygen sensor, and the processor configured to determine the target time or the target time interval comprises the processor configured to:
obtain a variation characteristic of the oxygen concentration detection value based on the oxygen concentration detection values at different times; and
determine whether the variation characteristic of the oxygen concentration detection value meets a preset variation condition, and determine the target time or the target time interval based on a time or time interval where the variation characteristic meets the preset variation condition,
wherein the interference of the interference gas with the oxygen concentration detection meeting the preset condition comprises the variation characteristic of the oxygen concentration detection value meeting the preset variation condition,
wherein the variation characteristic comprises a slope of the oxygen concentration detection value, and the preset variation condition comprises: a duration, during which the slope of the oxygen concentration detection value does not exceed a preset slope, reaches a preset first time length; or
the variation characteristic comprises a variance of the oxygen concentration detection value, and the preset variation condition comprises: the variance of the oxygen concentration detection value does not exceed a preset variance;
or
the processor configured to determine the target time or the target time interval comprises the processor configured to:
when the device parameter comprises a respiratory rate and a respiratory ratio set by the respiratory support device, determine a respiratory phase based on the respiratory rate and the respiratory ratio; or when the gas characteristic comprises at least one of: a flow rate detection value of a gas flowing through the ultrasonic oxygen sensor detected by the ultrasonic oxygen sensor, a flow rate detection value of a gas flowing through the ultrasonic oxygen sensor detected by a flow rate sensor, a flow rate detection value of the gas in the respiratory gas flow path detected by the flow rate sensor, or a pressure detection value of the gas in the respiratory gas flow path detected by a pressure sensor, determine a respiratory phase based on a waveform of the gas characteristic;
when the respiratory phase is an inhalation stage of a respiratory cycle, set a time within a time interval corresponding to a preset second time length before an end of inhalation, as the target time, or when the respiratory phase is an inhalation stage of the respiratory cycle, set a time interval corresponding to the preset second time length before an end of inhalation, as the target time interval,
wherein the target time or the target time interval is within the inhalation stage;
or
the gas characteristic comprises a flow direction of the gas in the respiratory gas flow path detected by a flow rate sensor, and the processor configured to determine the target time or the target time interval comprises the processor configured to:
when the flow direction changes from toward the patient to away from the patient, set a time, at which time a preset third time length has elapsed from when the flow direction changes from toward the patient to away from the patient, as a start time; and
set a time within a time interval, which time interval begins from the start time and lasts for a preset fourth time length, as the target time, or set a time interval, which time interval begins from the start time and lasts for the preset fourth time length, as the target time interval,
wherein a time when the flow direction changes from toward the patient to away from the patient is in a same respiratory cycle as the target time or the target time interval,
or
the processor configured to determine the target time or the target time interval comprises the processor configured to:
when the gas characteristic comprises a flow rate detection value of a gas flowing through the ultrasonic oxygen sensor detected by the ultrasonic oxygen sensor or a flow rate sensor, calculate, in an inhalation stage of a respiratory cycle, a volume of the gas flowing through the ultrasonic oxygen sensor in the inhalation stage based on the flow rate detection value; or when the device parameter comprises an output flow rate of the oxygen-containing gas, calculate, in an inhalation stage of a respiratory cycle, a volume of the gas flowing through the ultrasonic oxygen sensor in the inhalation stage based on the output flow rate of the oxygen-containing gas;
when the volume exceeds a preset volume, set a time, at which time a preset fifth time length has elapsed from when the volume exceeds the preset volume, as a start time; and
set a time within a time interval, which time interval begins from the start time and lasts for a preset sixth time length, as the target time, or set a time interval, which time interval begins from the start time and lasts for the preset sixth time length, as the target time interval.

7. The respiratory support device of claim 2, wherein when determining the target oxygen concentration detection value based on the gas characteristic and/or the device parameter, and optionally outputting the target oxygen concentration detection value, the processor is further configured to:
select one or more processing modes from a plurality of processing modes based on a preset rule; and
determine the target oxygen concentration detection value under the one or more processing modes based on the gas characteristic and/or the device parameter, and output the target oxygen concentration detection value,
wherein the preset rule comprises a preset priority rule for the plurality of processing modes and/or a preset accuracy judgment rule for the plurality of processing modes.

8. The respiratory support device of claim 7, wherein
when selecting one or more processing modes from the plurality of processing modes based on the preset rule, determining the target oxygen concentration detection value under the one or more processing modes based on the gas characteristic and/or the device parameter, and outputting the target oxygen concentration detection value, the processor is further configured to: select a first processing mode first, and if the target oxygen concentration detection value is capable of being determined under the first processing mode based on the gas characteristic and/or the device parameter, output the determined target oxygen concentration detection value; and when the target oxygen concentration detection value is not capable of being determined under the first processing mode, select a second processing mode, a third processing mode and/or a fourth processing mode, and if the target oxygen concentration detection value is capable of being determined under the selected processing mode based on the gas characteristic and/or the device parameter, output the determined target oxygen concentration detection value, wherein the first processing mode, the second processing mode, the third processing mode and the fourth processing mode are different from one another,
and/or
the gas characteristic comprises a flow direction of the gas in the respiratory gas flow path detected by a flow rate sensor; and when selecting one or more processing modes from the plurality of processing modes based on the preset rule, determining the target oxygen concentration detection value under the one or more processing modes based on the gas characteristic and/or the device parameter, and outputting the target oxygen concentration detection value, the processor is further configured to: after the flow direction changes from toward the patient to away from the patient, select a first processing mode, determine the target oxygen concentration detection value under the first processing mode based on the gas characteristic and/or the device parameter, and output the target oxygen concentration detection value.

9. The respiratory support device of claim 8, wherein the processor configured to select the second processing mode, the third processing mode and/or the fourth processing mode, and if the target oxygen concentration detection value is capable of being determined under the selected processing mode based on the gas characteristic and/or the device parameter, output the determined target oxygen concentration detection value, comprises the processor configured to:
when the device parameter comprises a respiratory ratio set by the respiratory support device and the respiratory ratio is greater than a preset ratio, and if the target oxygen concentration detection value is capable of being determined under the second processing mode and/or the fourth processing mode, output the target oxygen concentration detection value; when the respiratory ratio is less than the preset ratio, and if the target oxygen concentration detection value is capable of being determined under the third processing mode, output the target oxygen concentration detection value; or
when the gas characteristic comprises at least one of: a flow rate detection value of a gas flowing through the ultrasonic oxygen sensor detected by the ultrasonic oxygen sensor, a flow rate detection value of a gas flowing through the ultrasonic oxygen sensor detected by a flow rate sensor, a flow rate detection value of the gas in the respiratory gas flow path detected by the flow rate sensor, or a pressure detection value of the gas in the respiratory gas flow path detected by a pressure sensor, determine a time length of an exhalation stage in a respiratory cycle or a time length of an inhalation stage in a respiratory cycle based on a waveform of the gas characteristic; when the time length of the exhalation stage is greater than a preset first threshold or the time length of the inhalation stage is less than a preset second threshold, and if the target oxygen concentration detection value is capable of being determined under the second processing mode and/or the fourth processing mode, output the target oxygen concentration detection value; when the time length of the exhalation stage is less than the preset first threshold or the time length of the inhalation stage is greater than the preset second threshold, and if the target oxygen concentration detection value is capable of being determined under the third processing mode, output the target oxygen concentration detection value.

10. The respiratory support device of claim 7, wherein when selecting one or more processing modes from the plurality of processing modes based on the preset rule, determining the target oxygen concentration detection value under the one or more processing modes based on the gas characteristic and/or the device parameter, and outputting the target oxygen concentration detection value, the processor is further configured to:
determine the target oxygen concentration detection value under several of a first processing mode, a second processing mode, a third processing mode and a fourth processing mode based on the gas characteristic and/or the device parameter, and output the target oxygen concentration detection value, wherein the first processing mode, the second processing mode, the third processing mode and the fourth processing mode are different from one another,
wherein when determining the target oxygen concentration detection value and outputting the target oxygen concentration detection value, the processor is further configured to:
determine a plurality of candidate target oxygen concentration detection values under the several processing modes, and
select, based on a type of the interference gas, a corresponding candidate target oxygen concentration detection value from the plurality of candidate target oxygen concentration detection values as the target oxygen concentration detection value, and output the target oxygen concentration detection value.

11. The respiratory support device of claim 2, wherein when determining the target oxygen concentration detection value, and optionally outputting the target oxygen concentration detection value, the processor is further configured to:
set the oxygen concentration detection value at the target time as the target oxygen concentration detection value, and output the target oxygen concentration detection value; or
calculate an average value of the oxygen concentration detection values at a plurality of times in the target time interval, set the average value as the target oxygen concentration detection value, and output the target oxygen concentration detection value; or
when a plurality of target times or a plurality of target time intervals exist within a same respiratory cycle, select, based on a preset selection rule, one of the oxygen concentration detection values at the plurality of target times or in the plurality of target time intervals within the same respiratory cycle as the target oxygen concentration detection value, and output the target oxygen concentration detection value; or
when a plurality of target times or a plurality of target time intervals exist within a same respiratory cycle, determine the target oxygen concentration detection value based on the oxygen concentration detection values at the plurality of target times or in the plurality of target time intervals within the same respiratory cycle, and output the target oxygen concentration detection value,
wherein the processor configured to select, based on the preset selection rule, one of the oxygen concentration detection values at the plurality of target times or in the plurality of target time intervals within the same respiratory cycle as the target oxygen concentration detection value, and output the target oxygen concentration detection value, comprises the processor configured to:
select a minimum one of the oxygen concentration detection values at the plurality of target times or in the plurality of target time intervals within the same respiratory cycle as the target oxygen concentration detection value, and output the target oxygen concentration detection value,
wherein the processor configured to determine the target oxygen concentration detection value based on the oxygen concentration detection values at the plurality of target times or in the plurality of target time intervals within the same respiratory cycle, and output the target oxygen concentration detection value, comprises the processor configured to:
calculate an average value of the oxygen concentration detection values at the plurality of target times or in the plurality of target time intervals within the same respiratory cycle, set the average value as the target oxygen concentration detection value, and output the target oxygen concentration detection value.

12. The respiratory support device of claim **1,** wherein
the buffer member comprises a buffer gas path, and the buffer gas path is arranged at the rear end of the ultrasonic oxygen sensor and is configured to accommodate the interference gas through its own volume, such that the interference gas is unable to diffuse from the rear end of the ultrasonic oxygen sensor into the ultrasonic oxygen sensor; or
the isolation member comprises an on/off valve, the respiratory gas flow path is provided with a bypass, and both the on/off valve and the ultrasonic oxygen sensor are arranged in the bypass; the on/off valve is opened in an inhalation stage of a respiratory cycle and is closed in an exhalation stage of the respiratory cycle, such that the interference gas is unable to diffuse from the rear end of the ultrasonic oxygen sensor into the ultrasonic oxygen sensor.

13. The respiratory support device of claim 12, wherein
the buffer gas path comprises a pipeline with a volume no less than a preset value; or
the buffer gas path comprises an air container with a volume no less than the preset value;
preferably, the preset value is a value between 20-40 milliliters.

14. The respiratory support device of claim 1, wherein the respiratory support device is a ventilator or an anesthesia machine, preferably, the respiratory support device comprises a single-tube ventilator.

15. A method for detecting an oxygen concentration in a respiratory support device, wherein the respiratory support device comprises a respiratory gas flow path and an ultrasonic oxygen sensor, the respiratory gas flow path is configured to connect to a patient and deliver an oxygen-containing gas to the patient during a treatment time period to provide respiratory support, the respiratory gas flow path further optionally contains an interference gas that comprises at least part of an exhaled gas by the patient; the ultrasonic oxygen sensor is configured to detect an oxygen concentration of a gas in the respiratory gas flow path, and the gas in the respiratory gas flow path comprises at least the oxygen-containing gas and optionally the interference gas; the method comprising:
acquiring a gas characteristic of the gas in the respiratory gas flow path and/or a device parameter of the respiratory support device; and
determining a target oxygen concentration detection value based on the gas characteristic and/or the device parameter, and optionally outputting the target oxygen concentration detection value, wherein the target oxygen concentration detection value is obtained based on an oxygen concentration detection value of the ultrasonic oxygen sensor at a target time or in a target time interval where an interference of the interference gas with the oxygen concentration detection meets a preset condition.
